# EUROPEAN PATENT APPLICATION

(11) **EP 2 085 393 A1**
(43) Date of publication of application: **05.08.2009**
(21) Application number: 07828824.8
(22) Date of filing: 28.09.2007
(51) Int. Cl.: C07D 303/04, C07D 301/12

(54) **PROCESS FOR PRODUCTION OF EPOXY COMPOUND**

(30) Priority: 02.10.2006 JP 2006270441
(71) Applicant: Sumitomo Chemical Company, Limited, Tokyo 104-8260 (JP)
(72) Inventor: KAWABATA, Tomonori, Osaka 561-0802 (JP)
(74) Representative: Duckworth, Timothy John
(86) International application number: PCT/JP2007/069083
(87) International publication number: WO 2008/041654

(57) **Abstract**

An object of the present invention is to provide a method for producing an epoxy compound more efficiently from olefin, oxygen, and hydrogen. A method according to the present invention for producing an epoxy compound is a method for producing an epoxy compound from olefin, oxygen, and hydrogen. The method includes: (a) bringing a crystalline titanosilicate catalyst having an MFI structure into contact with a peroxide; and (b) reacting olefin, oxygen, and hydrogen with each other in a liquid phase in the presence of a noble metal catalyst and the crystalline titanosilicate catalyst obtained in the step (a).

## Description

### Technical Field

The present invention relates to a method for producing an epoxy compound from olefin, oxygen, and hydrogen.

### Background art

As a method for producing an epoxy compound from olefin, oxygen, and hydrogen, there has been known such a method that a mixed solvent of water and methanol is used for a reaction for catalytically producing propylene oxide from hydrogen, oxygen, and propylene wherein TS-1, which is a crystalline titanosilicate having an MFI structure, and palladium are catalysts (see, for example, Non-Patent Literature 1). There has been disclosed a method for improving the selectivity of propylene oxide to be produced in the reaction above. According to the method, a salt etc. is added during the reaction in order to improve the selectivity of propylene oxide (see, for example, Patent Literature 1).

### Citation List

Non-Patent Literature 1
   R. Meiers et al., Synthesis of Propylene Oxide from Propylene, Oxygen, and Hydrogen Catalyzed by Palladium-Platinum-containing Titanium silicate, Journal of Catalysis, 176, 376-386, (1998)
Patent Literature 1
   Japanese Translation of PCT International Application Publication No. 2002-511455 (Tokuhyo 2002-511455, date of publication: April 16, 2002)

### Summary of Invention

An object of the present invention is to provide a method for producing an epoxy compound more efficiently from olefin, oxygen, and hydrogen.

That is, the present invention relates to a method for producing epoxy compound, including: (a) bringing a crystalline titanosilicate catalyst having an MFI structure into contact with a peroxide; and (b) reacting olefin, oxygen, and hydrogen with each other in a liquid phase in the presence of a noble metal catalyst and the crystalline titanosilicate catalyst obtained in the step (a).

The present invention makes it possible to efficiently obtain an epoxy compound from olefin, oxygen, and hydrogen, with a fine selectivity, by a simple method utilizing that crystalline titanosilicate having the MFI structure, which has been treated by contact with the peroxide.

Additional objects, features, and strengths of the present invention will be made clear by the description below. Further, the advantages of the present invention will be evident from the following explanation in reference to the drawings.

### Description of Embodiments

Examples of a noble metal that is used in the present invention encompass palladium, platinum, ruthenium, rhodium, iridium, osmium, gold, and an alloy or mixture thereof. Preferable noble metals are palladium, platinum, and gold. Palladium is a more preferable noble metal. When palladium is used, a metal such as platinum, gold, rhodium, iridium, or osmium can be added and mixed therewith. A preferable additive metal is platinum.

The noble metals can be a compound thereof such as an oxide or a hydroxide thereof. The present invention may be arranged such that after a noble metal compound is placed in a reaction vessel, a part of or all of the metal compound is reduced under a reaction condition with hydrogen contained in reacting materials.

When used, a noble metal is generally supported on a carrier. The noble metal can be supported on a crystalline titanosilicate having an MFI structure, when used. Alternatively, the noble metal can be supported on (i) titanosilicate other than the crystalline titanosilicate having the MFI structure, (ii) a carrier other than titanosilicate, such as an oxide of silica, alumina, titania, zirconia, niobia or the like, a hydrate of such as niobic acid, zirconium acid, tungstic acid, titanic acid or the like, carbon, or (iii) a mixture thereof. In a case where a noble metal is supported on a carrier other than the crystalline titanosilicate having the MFI structure, a mixture of (i) the crystalline titanosilicate having the MFI structure and having been treated with a peroxide and (ii) the carrier supporting the noble metal can be used as catalysts. Carbon is a preferable carrier among the carriers except titanosilicate. There have been known carbon carriers such as activated carbon, carbon black, graphite, a carbon nanotube, etc.

A noble metal catalyst supported on a carrier may be prepared by the following method, for example. A noble-metal colloid solution is prepared by dispersing noble metal particles by use of a dispersant such as citric acid, polyvinyl alcohol, polyvinyl pyrrolidone, or sodium hexametaphosphate. Subsequently, an impregnation method or the like is performed with the noble-metal colloid solution so as to cause the noble metal to be supported on a carrier. Then, the resultant is calcinated in the presence of an inert gas. Alternatively, the noble metal catalyst supported on a carrier can be also prepared by the following method. A noble metal compound that serves as a noble metal source is supported on a carrier by the impregnation method etc. The noble metal compound is, for example, a nitrate salt of a noble metal such as palladium nitrate, a sulfate salt of palladium sulfate dihydrate etc., a halide of a noble metal such as palladium chloride, a palladium acetate carboxylate salt, an ammine complex such as Pd tetraammine chloride, or the like. Then, the resultant is reduced by a reducing agent. Alternatively, the noble metal catalyst supported on a carrier can be also prepared by the following method. A hydroxide of a noble metal is firstly prepared by use of an alkali such as sodium hydroxide. Then, the hydroxide is reduced in a liquid or a vapor phase by a reducing agent. Examples of the reducing agent for reducing the hydroxide in the liquid phase encompass hydrogen, hydrazine monohydrate, formaldehyde, sodium borohydride, etc. As a method for the reduction utilizing hydrazine monohydrate or formaldehyde, there has been known a method in which an alkali is added. Examples of the reducing agent for reducing the hydroxide in the vapor phase encompass hydrogen, ammonia, etc. The noble metal catalyst supported on a carrier can be also prepared by calcinating and reducing in the presence of hydrogen gas the carrier supporting the noble metal source. A preferable reduction temperature varies depending on a noble metal source supported on a carrier. A reduction temperature generally falls in a range from 0 °C to 500 °C. On the other hand, the noble metal catalyst supported on a carrier can be also prepared by the following method. An ammine complex of a noble metal such as Pd tetraammine chloride is supported by the impregnation method etc. Then, the resultant is reduced in the presence of an inert gas by ammonia gas to be generated in thermal decomposition. A reduction temperature varies depending on which type of an ammine complex of a noble metal is used. The reduction temperature is generally in a range from 100 °C to 500 °C, and preferably falls in a range from 200 °C to 350 °C in a case where Pd tetraammine chloride is used.

In any of the methods above, if necessary, it is possible to activate an obtained catalyst through heat treatment in an inert gas, ammonia gas, a vacuum, hydrogen, or an atmosphere. The noble metal catalyst supported on a carrier can be also prepared by the following method. After a compound such as an oxide or a hydroxide of a noble metal is placed in a reaction vessel, the compound is reduced under a reaction condition.

The resultant carrier supporting a noble metal usually contains the noble metal in a range from 0.01 wt% to 20 wt%, or preferably, in a range from 0.1 wt% to 5 wt%.

A weight ratio of a noble metal to the crystalline titanosilicate having the MFI structure (i.e., a ratio of an weight of the noble metal to a weight of the crystalline titanosilicate having the MFI structure) preferably falls in a range from 0.01 wt% to 100 wt%, or more preferably, in a range from 0.1 wt% to 20 wt%.

In the present invention, the crystalline titanosilicate having the MFI structure used herein refers to a crystalline titanosilicate having an MFI structure defined as a framework type code of IZA (International Zeolite Association). Specifically, TS-1 is exemplified as that crystalline titanosilicate having the MFI structure which is used in the present invention. It is also possible to use (i) Ti-MMM-1 (Microporous and Mesoporous Materials, volume 52, page 11 - 18, 2002) composed of a compatible phase of TS-1 and Ti-MCM-41, which is mesoporous titanosilicate, and (ii) a mixture in which TS-1 is supported on a carrier such as silica, provided that the effect of the present invention is not impaired.

Titanosilicate is a generic term referring to porous silicate (SiO₂) whose Si is partially substituted by Ti. Ti of titanosilicate is in the framework of SiO₂. This can be easily confirmed from an ultraviolet-visible absorption spectrum having a peak in a range from 210 nm to 230 nm. Alternatively, this can be easily confirmed through the measurement of a coordination number of Ti of titanosilicate by a Ti-K-edge XAFS analysis because Ti of TiO₂ usually has a coordination number of 6 whereas Ti of titanosilicate has a coordination number of 4.

It is known that it can be easily confirmed through the measurement of an X-ray diffraction pattern that titanosilicate has the MFI structure (e.g., Advances Catalysis, Volume 41, page 253-334). A general method for synthesizing the crystalline titanosilicate having the MFI structure is the following method. First, a titanium compound such as tetra-n-butyl-o-titanate or tetraethyl-o-titanate and a silicon compound such as tetraethyl-o-silicate are hydrolyzed by use of a surfactant such as tetra-n-propylammonium hydroxisde, which is a template or a structure-directing agent. Alcohol generated in the hydrolysis is removed as necessary. Then, the resultant is crystallized by hydrothermal synthesis etc. Finally, the surfactant is removed by calcination or extraction.

The crystalline titanosilicate having the MFI structure is treated with a peroxide before used as a catalyst. The treatment with the peroxide makes it possible to selectively synthesize propylene oxide. This allows efficient production of propylene oxide. The treatment in which the crystalline titanosilicate having the MFI structure and the peroxide are brought into contact with each other is carried out by, for example, bringing the crystalline titanosilicate having the MFI structure into contact with a solution containing the peroxide at an adequate concentration. Examples of the peroxide encompass organic peroxides such as ethylbenzene hydroperoxide, cumene hydroperoxide, t-butylhydroperoxide, and peracetic, and an inorganic peroxide such as hydrogen peroxide. The use of hydrogen peroxide is preferable because, in the event of decomposition of peroxide, the decomposition produces water and oxygen, which are easy to deal with. A suitable solvent for the peroxide varies depending on a type of the peroxide. In a case where hydrogen peroxide is used, a solvent may preferably be, but not limited to, water or a solvent used in synthesis of propylene oxide, for the sake of industrial convenience.

A concentration of the peroxide varies depending on the stability of a peroxide to be used. The treatment can be usually performed by use of a peroxide having a concentration of 0.0001 wt% or more. For example, in a case where hydrogen peroxide is used, the treatment can be usually performed by use of hydrogen peroxide having a concentration in a range from 0.0001 wt% to 50 wt%. A concentration of hydrogen peroxide preferably falls in a range from 0.01 wt% to 5 wt%. In a case where the solvent for a synthesis reaction of propylene oxide is used, the treatment is preferably performed by use of hydrogen peroxide having a concentration higher than that of hydrogen peroxide in a reaction solution in which propylene oxide is synthesized from water, oxygen, and propylene.

Methods for bringing the crystalline titanosilicate having the MFI structure into contact with hydrogen peroxide encompass the following methods, for example: (i) Before the reaction is carried out, an aqueous solution of hydrogen peroxide is added to the crystalline titanosilicate having the MFI structure, and then, the crystalline titanosilicate having the MFI structure is filtered out. Then, the crystalline titanosilicate having the MFI structure thus filtered out is used for the reaction. (ii) A reaction solvent containing hydrogen peroxide is flown through a catalytic mixture of the carrier supporting a noble metal and the crystalline titanosilicate having the MFI structure, so that the crystalline titanosilicate having the MFI structure is brought into contact with the peroxide in a reaction vessel. Then, the crystalline titanosilicate having the MFI structure thus treated is used for the reaction.

The crystalline titanosilicate having the MFI structure is brought into contact with the peroxide generally at a temperature of 0 °C or higher. An upper limit of the temperature varies depending on the stability of a peroxide solution to be used. The treatment is performed within a temperature range in which there is no danger posed by heat generated by self-decomposition of the peroxide.

In a case where hydrogen peroxide is used, the hydrogen peroxide treatment can be generally performed at a temperature in a range from 0 °C to 100 °C. The temperature preferably falls in a range from 0 °C to 60 °C. A treating time, which depends on the concentration of hydrogen peroxide, usually falls in a range from 10 minutes to 5 hours. The treating time preferably falls in a range from 1 hour to 3 hours.

The reaction of the present invention is generally carried out in the liquid phase of water, an organic solvent, or the mixture thereof. Examples of the organic solvent encompass alcohol, ketone, nitrile, ether, aliphatic hydrocarbon, aromatic hydrocarbon, halogenated hydrocarbon, ester, glycol, and the mixture thereof. Examples of a preferable organic solvent encompass linear or branched-chain saturated aliphatic nitrile, and aromatic nitrile. Examples of the nitrile compounds encompass C₂-C₄ alkyl nitriles such as acetonitrile, propionitrile, isobutyronitrile, and butyonitrile; and benzonitrile. Acetonitrile is preferable.

A weight ratio of water to an organic solvent usually falls in a range from 90:10 to 0.01:99.99. The weight ratio preferably falls in a range from 50:50 to 0.01:99.99. An excessively high ratio of water causes a reaction of an epoxy compound with water in some cases. As a result of the reaction, the epoxy compound undergoes ring-opening deterioration. This can decrease the selectivity of the epoxy compound. Conversely, an excessively high ratio of the organic solvent increases the costs of recovery of the solvent.

In the method of the present invention, it is effective to adopt a method in which a salt of ammonium, alkylammonium, or alkylarylammonium, the crystalline titanosilicate having the MFI structure, and the noble metal solvent are added together in the reaction solvent, because the method can improve the use efficiency of hydrogen by preventing decrease in catalyst activity, and increasing the catalyst activity. The salt of ammonium, alkylammonium, or alkylarylammonium is added generally in an amount in a range from 0.001 mmol/kg to 100 mmol/kg per unit weight of the solvent (or a total weight of the mixture of water and an organic solvent).

Examples of the salt of ammonium, alkylammonium, or alkylarylammonium encompass a salt of a cation selected from the group consisting of ammonium, alkylammonium, and alkylarylammonium, with an anion selected from the group consisting of sulfate ion, hydrogen sulfate ion, carbonate ion, hydrogen carbonate ion, phosphate ion, hydrogen phosphate ion, dihydrogen phosphate ion, hydrogen pyrophosphate ion, pyrophosphate ion, halogen ion, nitrate ion, hydroxide ion, and C₁-C₁₀ carboxylate ions.

Examples of the C₁-C₁₀ carboxylate ion encompass formate ion, acetate ion, propionate ion, butyrate ion, valerate ion, caproate ion, caprylate ion, and caprate ion.

Examples of the alkylammonium encompass tetramethylammonium, tetraethylammonium, tetra-n-propylammonium, tetra-n-butylammonium, and cetyltrimethylammonium.

Examples of a preferable salt of ammonium, alkylammonium, or alkylarylammonium encompass an ammonium salt of an inorganic acid such as ammonium sulfate, ammonium hydrogen sulfate, ammonium carbonate, ammonium hydrogen carbonate, diammonium hydrogen phosphate, ammonium dihydrogen phosphate, ammonium phosphate, ammonium hydrogen pyrophosphate, ammonium pyrophosphate, ammonium chloride, or ammonium nitrate, and an ammonium salt of a C₁-C₁₀ carboxylic acid such as ammonium acetate. The ammonium salt of ammonium dihydrogen phosphate is preferable.

Olefin that is used in the present invention refers to hydrocarbon having more than one carbon-carbon double bond. Examples of the olefin that is used in the present invention encompass an aliphatic olefin such as ethylene, propylene, 1-butene, 2-butene, 1-pentene, 1-hexene, or 2-hexene; a cyclic olefin such as cyclopentene or cyclohexene; a diolefin such as butadiene; and an olefin having an aromatic ring, such as styrene.

The present invention is suitably applicable to a method for producing epoxide from a C₂-C₆ olefin such as ethylene, propylene, 1-butene, 2-butene, 1-pentene, 1-hexene, 2-hexene, cyclopentene, cyclohexene, or butadiene. Especially, the present invention is suitably applicable to a method for producing propylene oxide from propylene.

The reaction of the present invention may be carried out by continuous fluidized bed reaction, continuous slurry complete mixing reaction, etc. A ratio of partial pressures of oxygen to hydrogen, which are supplied to the reaction vessel, usually falls in a range from 1:50 to 50:1. A preferable ratio of the partial pressures of oxygen to hydrogen falls in a range from 1:2 to 10:1. An excessively high ratio of the partial pressures of oxygen to hydrogen (oxygen/hydrogen) would decrease generation speed of epoxide. An excessively low ratio of the partial pressures of oxygen to hydrogen (oxygen/hydrogen) would decrease the selectivity of an epoxy compound because an amount of paraffin as a byproduct increases. The reaction may be carried out with oxygen and hydrogen diluted with a diluent gas. Examples of the diluent gas encompass nitrogen, argon, carbon dioxide, methane, ethane, and propane. A concentration of the diluent gas is not particularly limited. Oxygen and hydrogen are diluted as necessary, before used in the reaction.

Examples of a source of oxygen encompass an oxygen gas, air, etc. The oxygen gas may be an oxygen gas produced by a pressure swing method, which is advantageous in cost. Alternatively, as necessary, the oxygen gas may be a high-purity oxygen gas produced by cryogenic separation etc.

A reaction temperature of the reaction is generally in a range from 0 °C to 150 °C, or preferably, in a range from 40 °C to 90 °C. An excessively low reaction temperature slows the reaction rate. On the other hand, an excessively high reaction temperature increases a byproduct generated by a side reaction.

A reaction pressure is not particularly limited, but is generally in a range of gauge pressures from 0.1 MPa to 20 MPa, or preferably, in a range of gauge pressures from 1 MPa to 10 MPa. An excessively low reaction pressure causes insufficient dissolution of a raw material gas. This slows the reaction rate. An excessively high reaction pressure increases the cost of apparatuses involved in the reaction. Collection of the epoxy compound, which is a product of the present invention, can be carried out through an ordinary distillation.

### [Examples]

The following describes examples of the present invention. However, the present invention is not limited to the examples.

### (Example 1)

TS-1 used for the reaction of the present invention was prepared in accordance with a method described in Journal of Catalysis, 130, (1991), 1-8. By measuring an X-ray diffraction pattern, it was confirmed that obtained powder was TS-1. According to ICP emission spectrometry, the TS-1 contained a titanium content by 1.3 wt%.

### <Catalyst A>

A Pd/carbon black (CB) catalyst used for the reaction was prepared in accordance with a method described in US Patent Application Publication 2005-0014636. 0.56 mmol of palladium chloride, 0.026 mmol of platinum chloride, sodium polyacrylate (molecular weight: 1200, 1.27 mmol), and 500 mL of an aqueous solution containing 30 mmol of hydrogen chloride were mixed in a 500 mL recovery flask, and stirred for 1 hour at room temperature in an air atmosphere. Hydrogen gas was introduced into the mixture solution at a rate of 100 mL/min for 20 minutes at room temperature. Thereby, a Pd colloid was formed. 6g of CB (SEAST 9, Tokai Carbon Co., Ltd.), which was commercially available, was added in the colloid solution, and stirred for 8 hours. Then, water was removed by use of a rotary evaporator. Further, the resultant was dried in a vacuum at a temperature of 50 °C for 12 hours. The resultant catalyst precursor powder was calcinated at a temperature of 300 °C in the presence of a nitrogen atmosphere for 6 hours. Thus, a Pd/CB catalyst was obtained. According to ICP emission spectrometry, the Pd/CB catalyst contained a palladium content by 0.9 wt% and a platinum content by 0.1 wt%.

0.133 g of a TS-1 catalyst was treated at ambient temperature for 1 hour by use of 100 g of that solution of water and methanol (weight ratio: 20/80), which contained 0.1 wt% hydrogen peroxide. The resultant was washed by 500 mL of water, and then, filtered. Thus obtained was a TS-1 catalyst treated with hydrogen peroxide.

A 0.5 L autoclave was used as a reaction vessel for the reaction. A raw material gas containing propylene, oxygen, hydrogen, and nitrogen at volume ratios of 4/3/8/85, respectively, was supplied into the autoclave at a rate of 16 L/h. Concurrently, a solution containing water and methanol at a weight ratio of 20/80 was supplied into the autoclave at a rate of 108 mL/h. A liquid phase of the reaction mixture was removed out of the reaction vessel via a filter. Thus, a continuous reaction was caused under the following conditions: a temperature of 60 °C; a gauge pressure of 0.8 MPa; and a residence time of 90 minutes. Meanwhile, 131 g of a reaction solvent, 0.133 g of the TS-1 treated with hydrogen peroxide, and 0.03 g of Pd/CB were in the reaction mixture in the reaction vessel.

A liquid phase and a vapor phase extracted after 5 hours from the start of the reaction were analyzed by gas chromatography. As a result, a generation activity of propylene oxide (PO) per unit weight of TS-1 was 6.55 mmol-PO/g-TS-1·h. A selectivity based on propylene was 43%.

### (Example 2)

The same operation as that of Example 1 was performed except that a TS-1 treated with a solution of water and acetonitrile (weight ratio: 20/80) with 0.1 wt% hydrogen peroxide was used instead of the solution of water and methanol (weight ratio: 20/80) with 0.1 wt% hydrogen peroxide. A liquid phase and a vapor phase extracted after 5 hours from the start of the reaction were analyzed by gas chromatography. As a result, a generation activity of propylene oxide per unit weight of TS-1 was 4.82 mmol-PO/g-TS-1·h. A selectivity based on propylene was 36%.

### (Comparative Example 1)

The same operation as that of Example 1 was performed except that an untreated TS-1 was used instead of the TS-1 treated with the solution of water and methanol (weight ratio: 20/80) with 0.1 wt% hydrogen peroxide. A liquid phase and a vapor phase extracted after 5 hours from the start of the reaction were analyzed by gas chromatography. As a result, a generation activity of propylene oxide per unit weight of TS-1 was 6.45 mmol-PO/g-TS-1·h. A selectivity based on propylene was 27%.

The concrete embodiment and examples of implementation discussed in the foregoing detailed explanation serve solely to illustrate the technical details of the present invention, which should not be narrowly interpreted within the limits of such concrete embodiment and examples, but rather may be applied in many variations within the spirit of the present invention, provided such variations do not exceed the scope of the patent claims set forth below.

### Industrial Applicability

The present invention is applicable to all the industries in which an epoxy compound is dealt with.

## Claims

1. A method for producing an epoxy compound, comprising:
(a) bringing a crystalline titanosilicate catalyst having an MFI structure into contact with a peroxide; and
(b) reacting olefin, oxygen, and hydrogen with each other in a liquid phase in the presence of a noble metal catalyst and the crystalline titanosilicate catalyst obtained in the step (a).

2. The method for producing an epoxy compound as set forth in claim 1, wherein the peroxide is hydrogen peroxide.

3. The method for producing an epoxy compound as set forth in claim 1 or 2, wherein the noble metal catalyst is palladium, ruthenium, rhodium, iridium, osmium, gold, or, an alloy or mixture thereof.

4. The method for producing an epoxy compound as set forth in claim 3, wherein the noble metal catalyst is palladium.

5. The method for producing an epoxy compound as set forth in any one of claims 1 through 4, wherein the olefin is a C₂-C₆ olefin.

6. The method for producing an epoxy compound as set forth in claim 5, wherein the olefin is propylene.
